# EUROPÄISCHE PATENTSCHRIFT

(11) **EP 2 215 046 B1**
(45) Veröffentlichungstag und Bekanntmachung des Hinweises auf die Patenterteilung: **17.08.2011**
(21) Anmeldenummer: 08841980.9
(22) Anmeldetag: 14.10.2008
(51) Int. Cl.: C07C 209/16, C07C 211/07

(54) **VERFAHREN ZUR HERSTELLUNG EINES PRIMÄREN AMINS MIT TERTIÄREM ALPHA-C-ATOM DURCH UMSETZUNG EINES TERTIÄREN ALKOHOLS MIT AMMONIAK**
PROCESS FOR PREPARING A PRIMARY AMINE WITH A TERTIARY ALPHA CARBON ATOM BY REACTING A TERTIARY ALCOHOL WITH AMMONIA
PROCÉDÉ DE PRODUCTION D'UNE AMINE PRIMAIRE AYANT UN ATOME DE CARBONE ALPHA TERTIAIRE PAR MISE EN RÉACTION D'UN ALCOOL TERTIAIRE AVEC DE L'AMMONIAC

(30) Priorität: 24.10.2007 EP 07119205; 02.09.2008 EP 08163456
(43) Veröffentlichungstag der Anmeldung: 11.08.2010
(73) Patentinhaber: BASF SE, 67056 Ludwigshafen (DE)
(72) Erfinder: SIGL, Marcus, 68165 Mannheim (DE); HEIDEMANN, Thomas, 68519 Viernheim (DE)
(86) Internationale Anmeldenummer: PCT/EP2008/063787
(87) Internationale Veröffentlichungsnummer: WO 2009/053275

(56) Entgegenhaltungen:
- US-A- 3 384 667
- US-A- 5 780 680
- DATABASE WPI Week 199226 Thomson Scientific, London, GB; AN 1992-212009 XP002515316 & JP 04 139156 A (MITSUI TOATSU CHEM INC) 13. Mai 1992 (1992-05-13) in der Anmeldung erwähnt
- SRINIVAS N ET AL: "Shape-Selective Synthesis of Collidines over Modified Zeolites" JOURNAL OF CATALYSIS, ACADEMIC PRESS, DULUTH, MN, US, Bd. 208, Nr. 2, 10. Juni 2002 (2002-06-10), Seiten 332-338, XP004465785 ISSN: 0021-9517

## Beschreibung

Die vorliegende Erfindung betrifft ein Verfahren zur Herstellung eines primären Amins mit tertiärem alpha-C-Atom durch Umsetzung eines tertiären Alkohols mit Ammoniak in Gegenwart eines Heterogen-Katalysators.

Primäre Amine mit tertiärem alpha-C-Atom finden vielfältige Verwendung als chemische Zwischenprodukte.

Z.B. kann 1-Adamantanylamin nach seiner Umsetzung zu 1-Adamantyltrimethylammonium-hydroxyd als Templat für die Hydrothermalsynthese von Chabazit verwendet werden.

Weiterhin kann z.B. tertiär-Butylamin nach seiner Umsetzung zu N-tertiär-Butyl-2-benzothiazylsulfenamid als Prozesschemikalie für die Gummiherstellung verwendet werden.

Die für das erfindungsgemäße Verfahren benötigten Edukte, die entsprechenden tertiären Alkohole, sind kommerziell verfügbar.

Z.B. wird das für die Herstellung von tertiär-Butylamin benötigte tertiär-Butanol vorzugsweise aus Isobuten-haltigen Kohlenwasserstoffströmen gewonnen. Hierfür werden technische C4-Fraktionen an sauren Katalysatoren, vorzugsweise 65%-ige Schwefelsäure, mit Wasser umgesetzt.

Zudem fällt tertiär-Butanol auch als Koppelprodukt bei der Herstellung von Propylenoxid an.

Technische Verfahren zur Herstellung eines primären Amins mit tertiärem alpha-C-Atom durch Umsetzung eines tertiären Alkohols beruhen auf der Ritter-Reaktion (vgl. Römpp, Lexikon Chemie, 10. Auflage, Thieme Verlag, Herausgeber: J. Falbe, M. Regitz, Band 5 (1998), Seite 3836). Bei diesem Verfahren wird tertiärer Alkohol in Gegenwart einer stöchiometrischen Menge an Schwefelsäure mit Blausäure umgesetzt. Anschließend wird mit Natronlauge neutralisiert und es bilden sich neben dem Amin Natriumsulfat und Natriumformiat als Nebenprodukte.

Die Herstellung von tertiär-Butylamin aus tertiär-Butanol nach dem Ritter-Verfahren sind unter anderem beschrieben in DE-B1-22 36040 (BASF AG) und EP-A1-50 870 (Degussa).

Eindeutige Nachteile der Ritter-Chemie sind die Verwendung von giftiger Blausäure als Stickstoffquelle und der hohe Salzanfall. Man ist demnach bestrebt, Blausäure durch Ammoniak als Stickstoffquelle zu ersetzen.

Die Umsetzung von Alkoholen mit Ammoniak kann prinzipiell auf drei Arten erfolgen:
Reduktive Aminierung an Katalysatoren, die Wasserstoff übertragen können und dafür Übergangsmetalle wie Ni, Co, Pd oder Pt enthalten.
Formselektive Aminierung an Katalysatoren, die Mikroporen enthalten, wie Zeolithe, Molekularsiebe oder kristalline Alumosilikate.
Saure Aminierung an Katalysatoren, die saure Zentren aufweisen, wie Aluminiumoxid oder Alumosilikate (= gemischte Aluminium- und Siliciumoxide).

Verfahren der reduktiven Aminierung sind zur Herstellung von primären Aminen mit tertiärem alpha-C-Atom weniger geeignet, da aufgrund des tertiären alpha-C-Atoms die Ausbildung einer Imin-Zwischenstufe nicht möglich ist.

Für die formselektive Aminierung ist nur eine Schrift bekannt, in der die Umsetzung tertiäre Alkohole beschrieben wird (JP-A-041 39156, s.u.).

Verfahren zur sauren Aminierung sind noch nicht für die Umsetzung von tertiären Alkoholen mit Ammoniak vorbeschrieben.

US 3,384,667 (Mobil Oil Corp.) betrifft die Herstellung von Aminen aus aliphatischen und aromatischen Alkoholen, wie n-Butanol und Phenol (siehe die Beispiele), und Ammoniak in Gegenwart von bestimmten kristallinen Aluminosilikat-Katalysatoren. Die Katalysatoren (Zeolithe) sind mikroporös, mit einem Porendurchmesser im Bereich von 5 bis 10 Angström (Spalte 2, Zeilen 3-14).

US 4,205,012 (ICI Ltd.) beschreibt die Verwendung von FU-1-Zeolithen als Katalysatoren bei der Herstellung von insbesondere Methylaminen.

EP-A-180 983 (Air Products and Chemicals, Inc.) lehrt die Verwendung bestimmter dealuminierter Zeolithe, insbesondere dealuminierte H-Mordenite, als Katalysatoren bei der Herstellung von insbesondere Ethylaminen.

EP-A-324 267 (UOP) betrifft die Herstellung von Alkylaminen aus Alkoholen, insbesondere die selektive Herstellung von Monomethylamin aus Methanol (Seite 2, Zeilen 9 - 10), unter Verwendung von nicht-zeolithischen Molsieben.

Die nicht-zeolithischen Molsiebe werden dabei genauer als kristalline Strukturen, die mindestens die Elemente Al, Si und P enthalten, charakterisiert (Seite 4, Zeilen 55 - 57). Die meisten Strukturen sind explizit als mikroporös beschrieben (s. z.B. Seite 8, Zeilen 12-14; Seite 9, Zeilen 43-44; Seite 11, Zeilen 46-47; Seite 20, Zeilen 37 - 38).

Ein Verfahren zur Herstellung von primären Aminen mit tertiärem alpha-C-Atom aus den entsprechenden Alkoholen ist beschrieben in JP-A-041 39156 (Mitsui Toatsu Chem. Inc.). Hier wird die Verwendung von bestimmten kristallinen Silicoaluminophosphat-Katalysatoren gelehrt. Die Katalysatoren sind mikroporös.

Nachteil der im Stand der Technik beschriebenen mikroporösen und kristallinen Katalysatoren ist, dass deren Herstellung in der Regel aufwendig und kostspielig ist, da organische Templatmoleküle verwendet werden müssen, um die gewünschten Strukturen im Laufe der Hydrothermalsynthesen zu erhalten, welche nach der Kristallisation aus dem Material heraus gebrannt werden müssen.

Der vorliegenden Erfindung lag die Aufgabe zugrunde, unter Überwindung eines oder mehrerer Nachteile des Stands der Technik, ein verbessertes wirtschaftliches Verfahren zur Herstellung von primären Aminen mit tertiärem alpha-C-Atom aufzufinden.

Demgemäß wurde ein Verfahren zur Herstellung eines primären Amins mit tertiärem alpha-C-Atom durch Umsetzung eines tertiären Alkohols mit Ammoniak in Gegenwart eines Heterogen-Katalysators gefunden, welches dadurch gekennzeichnet ist, dass man die Umsetzung in Gegenwart eines nicht-mikroporösen nicht-zeolithischen Alumosilikats als Katalysator durchführt, wobei das Alumosilikat ein molares Al/Si-Verhältnis im Bereich von 0,1 bis 30 aufweist.

Insbesondere eignet sich das erfindungsgemäße Verfahren zur Herstellung eines primären Amins mit tertiärem alpha-C-Atom der Formel RR'R"C-NH₂ durch Umsetzung eines tertiären Alkohols der Formel RR'R"C-OH, wobei R, R' und R" organische Reste mit jeweils mindestens einem Kohlenstoffatom darstellen.

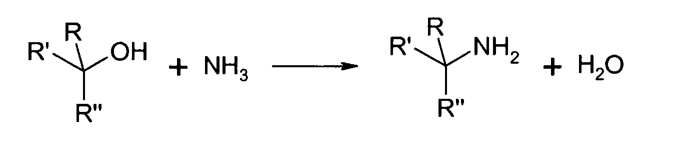

Ganz besonders bevorzugt wird mit dem erfindungsgemäßen Verfahren tertiär-Butylamin aus 2-Methyl-2-propanol, 1-Adamantylamin aus 1-Adamantanol und 2-Methyl-2-butylamin aus 2-Methyl-2-butanol hergestellt.

Beim im erfindungsgemäßen Verfahren eingesetzten Katalysator handelt es sich um einen nicht-mikroporösen nicht-zeolithischen Katalysator. Der Katalysator ist amorph (= nicht-kristallin).

Der Begriff "nicht-mikroporös" ist vorliegend derart definiert, dass der Katalysator keine Poren mit einem Durchmesser kleiner 0,8 nm, bevorzugt kleiner 1,2 nm, besonders bevorzugt kleiner 1,5 nm und ganz besonders bevorzugt kleiner 2,0 nm aufweist. Nicht-mikroporöse Alumosilikat-Katalysatoren werden z.B. in großtechnischen Anlagen zur Herstellung von Methylaminen eingesetzt.

Erfindungsgemäß wurde erkannt, dass nicht-mikroporöse Alumosilikat-Katalysatoren vorteilhaft für die saure Aminierung zur Herstellung von primären Aminen mit tertiärem alpha-C-Atom, z.B. der allgemeinen Formel RR'R"C-NH₂, aus den entsprechenden tertiären Alkoholen, eingesetzt werden können. Sie weisen gegenüber formselektiven (zeolithischen) Aminierungskatalysatoren Vorteile in den Herstellkosten auf. Überraschenderweise liefert das erfindungsgemäße Verfahren das Verfahrensprodukt in sehr hoher Selektivität. Folgeprodukte wie sekundäre Amine oder Nebenprodukte wie Ether, die durch Kondensation zweier Ausgangsalkohole entstehen, werden nur in sehr geringen Mengen gebildet.

Die Reste R, R' und R" stellen organische Reste dar, die jeweils mindestens ein Kohlenstoffatom besitzen.

Bevorzugt bedeuten R, R' und R" unabhängig voneinander lineare oder verzweigte Alkylreste mit jeweils 1 bis 16 Kohlenstoffatomen, bevorzugt jeweils 1 bis 6 C-Atomen, oder Cycloalkylreste mit jeweils 5 bis 7 Kohlenstoffatomen. Die Reste R und R' und/oder R" können auch zu einem 5- bis 12-gliedrigen, bevorzugt 6-gliedrigen, Ring aus C-Atomen geschlossen sein.

Beispiele für die Reste R, R' und R" sind (unabhängig voneinander): Methyl, Ethyl, n-Propyl, iso-Propyl, n-Butyl, iso-Butyl, n-Pentyl, n-Hexyl, n-Heptyl, n-Octyl, n-Nonyl, n-Decyl, Cyclopentyl, Cyclohexyl.

Das erfindungsgemäße Verfahren kann wie folgt durchgeführt werden.

Die Herstellung der Amine erfolgt durch Umsetzung von Ammoniak mit dem entsprechenden tertiären Alkohol bei erhöhtem Druck und erhöhter Temperatur an den amorphen Silica-Alumina-Katalysatoren (Mischformen von Aluminium- und Siliziumoxid). Wahlweise kann das Feedgemisch auch Wasser und/oder das herzustellende Amin enthalten.

Für die saure Aminierung gemäß dem erfindungsgemäßen Verfahren ist ein kontinuierlich durchströmtes Festbett-Reaktorsystem bevorzugt. Geeignete Reaktoren sind z.B. Rohrreaktoren, Rohrbündelreaktoren, Hordenreaktoren, Wickelreaktoren oder Wendelreaktoren. Die Umsetzung der tertiären Alkohole zu den Aminen ist exotherm. Die Temperaturkontrolle kann wie üblich mit Wärmetauschern durchgeführt werden. Die Wärmetauscher können im Reaktor (intern) angebracht sein oder außerhalb des Reaktors (extern). Bevorzugt wird die Reaktion in einem adiabaten Reaktorsystem ausgeführt.

Um den Katalysator in einem kontinuierlich durchströmten Festbett-Reaktorsystem zu verwenden, wird der Katalysator bevorzugt als Formkörper eingesetzt. Die Geometrie des Formkörpers wird so gewählt, dass der Druckverlust im Reaktor möglichst gering ist.

Die Verweilzeit im Reaktor wird bevorzugt so eingestellt, dass am heterogenen Katalysator das thermodynamische Gleichgewicht näherungsweise erreicht wird. Dies führt typischerweise zu Katalysatorbelastungen im Bereich von 0,1 bis 2,0 kg(tertiärer Alkohol) • kg(Katalysator)⁻¹ • h⁻¹, insbesondere im Bereich von 0,2 bis 1,5 kg(tertiärer Alkohol) • kg(Katalysator)⁻¹ • h⁻¹, ganz besonders im Bereich von 0,4 bis 1,0 kg(tertiärer Alkohol) • kg(Katalysator)⁻¹ • h⁻¹.

Das molare Verhältnis Ammoniak zu tertiärem Alkohol im Feedgemisch liegt bevorzugt im Bereich von 0,6 bis 12, besonders im Bereich von 0,8 bis 4,0, ganz bevorzugt im Bereich von 1 bis 3, besonders bevorzugt im Bereich von 1,1 bis 2,5.

Die Umsetzung wird bevorzugt bei einer Temperatur im Bereich von 220 bis 500 °C, insbesondere 230 bis 300 °C, z. B. 250 bis 290 °C, durchgeführt.

In einer Verfahrensvariante wird die Umsetzung ganz bevorzugt bei einer Temperatur im Bereich 300 bis 475°C, besonders bevorzugt 325 bis 450°C, durchgeführt.

In einer weiteren bevorzugten Ausführungsform wird die Umsetzung unter isothermen Bedingungen durchgeführt.

Der Absolutdruck der Umsetzung liegt bevorzugt im Bereich von 5 bis 400 bar, ganz bevorzugt im Bereich von 10 bis 250 bar und besonders bevorzugt im Bereich von 20 bis 100 bar.

Der Umsatz an Alkohol liegt bevorzugt bei > 10 %, besonders bevorzugt zwischen 20 % und 99,9 %, insbesondere zwischen 30 % und 99 %.

Die Selektivität der Umsetzung zur Bildung des primären Amins mit tertiärem alpha-C-Atom als Verfahrensprodukt (bezogen auf umgesetzten Alkohol) liegt besonders bei > 90 %, ganz besonderes bei > 95 %.

Nicht umgesetzter Ammoniak und/oder Alkohol kann nach Abtrennung des Amins zurückgeführt und erneut über den Katalysator geleitet werden. Dieser Rückführstrom kann auch Wasser und/oder Amin enthalten, bevorzugt in geringen Mengen kleiner 10 Gew.-%, bevorzugt kleiner 5 Gew.-%.

Für das erfindungsgemäße Verfahren geeignete amorphe Silica-Alumina-Katalysatoren, d.h. nicht-mikroporöse nicht-zeolithische Alumosilikate, wobei das Alumosilikat ein molares Al/Si-Verhältnis im Bereich von 0,1 bis 30 aufweist, können beispielsweise ausgewählt werden, aus den in den folgenden Dokumenten, die die Herstellung von Methylaminen betreffen, beschriebenen Katalysatoren: WO-A-2007/036478 (BASF AG) beschreibt einen Formkörper enthaltend ein Alumosilikat und Aluminiumoxid, wobei der Formkörper ein molares Al/Si-Verhältnis im Bereich von 10 bis 30 und für Poren mit einem Durchmesser von größer 1 nm eine zumindest bimodale Porenverteilung aufweist, wobei das Volumen der Poren des Formkörpers mit einem Durchmesser von größer 10 nm mindestens 40 % des gesamten Porenvolumes des Formkörpers entspricht.

US 1,875,747 (Martin et al.) offenbart Alumosilikat-Katalysatoren. Das Alumosilikat kann als synthetischer Feststoff oder in Form von Tonerde eingesetzt werden.

In EP-A-64 380 (DuPont) wird ein Katalysator beschrieben welcher durch die Behandlung eines Alumosilikats mit den Hydroxidsalzen des Natriums, Kaliums, Lithiums, Bariums oder Strontiums erhalten wird, wobei der Anteil an Na, K, Li, Ba oder Sr zwischen 0,1 und 6 Gew.-% liegt.

In DD-A-149 213 (VEB Leuna) werden dehydratisierend wirkende Katalysatoren, die aktives Aluminiumoxid enthalten, offenbart, wobei ein auf der Basis von Kaolin und Pseudoböhmit hergestellter Katalysator beschrieben wird, der neben Aluminiumoxid 12 bis 18 Gew.-% Siliciumdioxid enthält, dessen Gesamtporenvolumen größer als 0,5 ml/g ist, wobei der Anteil der Poren mit einem Durchmesser kleiner als 4 nm mindestens 30 % und größer als 15 nm höchstens 10 % beträgt und seine Korngröße bzw. Wandstärke kleiner 4 mm ist. Der Anteil von SiO₂ zwischen 12 und 18 % entspricht einem molaren Al/Si-Verhältnis zwischen 5,4 und 8,6.

In EP-A-62 428 (= US 4,370,503) (DuPont) wird ein Katalysator bestehend aus 88 bis 99 Gew.-% Alumina und 1 bis 13 Gew.-% Silica beschrieben. Die Gewichtsverteilungen an Silica und Alumina entsprechen einem molaren Al/Si-Verhältnis von 7,9 bis 116,7. Der Katalysator liegt üblicherweise als Tablette mit einem Durchmesser und/oder Länge von 3 bis 13 mm vor. Das Porenvolumen der Tablette liegt zwischen 0,2 und 0,8 ml/g, die BET-Oberfläche zwischen 100 und 250 m²/g.

In DD-A-108 275 (Becker et al.) werden Katalysatoren, bestehend aus Aluminiumoxid und/oder Alumosilikaten beschrieben, wobei der Katalysator in Form eines Hohlstranges verwendet wird, dessen Gesamtdurchmesser 3 bis 10 mm und dessen Hohlraumdurchmesser 1 bis 5 mm betragen, ein Gesamtporenvolumen von mindestens 30 % Poren mit einem Durchmesser größer als 100 Å, eine Oberfläche von mindestens 130 m²/g und eine Azidität von höchstens 2,0 x 10⁻⁵ mol NH₃/g besitzt.

In DE-A-1 543 731 (Leonard) werden Katalysatoren gelehrt, wobei der Katalysator aus einer Silicagelbasis besteht, auf welches aktives Aluminiumoxid und Spuren von Metallsalzvermittlern aufgetragen sind, und der Katalysator vor Benutzung durch eine Behandlung mit Wasserdampf von 1 - 50 Atmosphären teilweise deaktiviert wird. Der Katalysator enthält üblicherweise zwischen 12 und 13 Gew.-% Al₂O₃. Durch die Dampfbehandlung wird die Gesamtoberfläche des Katalysators auf 90 ± 20 m²/g reduziert und das Porenvolumen auf 0,34 ± 0,10 ml/g und der Porendurchmesser auf 74 ± 10 Å eingestellt.

Von besonderem technischen Interesse und daher bevorzugt sind Katalysatoren, die eine lange Standzeit aufweisen. Dies wird bei den im erfindungsgemäßen Verfahren eingesetzten Katalysatoren zum einen erzielt, indem die Azidität derart eingestellt wird, dass eine Aktivierung der Edukte durch Komplexbildung mit dem Aktivzentrum möglich ist, jedoch die Komplexe nicht so stabil sind, dass sich Coke und/oder Cokevorstufen bilden bzw. die Aktivzentren deaktivieren. Zum anderen wird die Porosität derart eingestellt, dass eine unter den gegebenen Reaktionsbedingungen ausreichende Diffusion der Edukte und Produkte von und zu den Aktivzentren sowie vom Reaktionsmedium in und aus den Poren des Katalysators möglich ist. Solche Katalysatoren sind beispielsweise in WO-A-2007/036478 (BASF AG; s.o.) beschrieben.

Die Herstellung eines geeigneten Katalysators erfolgt bevorzugt nach einem Verfahren, das durch die folgenden Schritte gekennzeichnet ist:
(I) Herstellen eines Gemischs, enthaltend eine SiO₂-Quelle, eine Al₂O₃-Quelle und ein Bindemittel,
(II) Vermischen und Verdichten des Gemischs,
(III) Verformen des verdichteten Gemischs unter Erhalt eines Formkörpers,
(IV) Calcinieren des Formkörpers.

Für die Herstellung des Gemisches nach Schritt (I) werden bevorzugt Tone verwendet und können insbesondere bevorzugt Schichtsilikate aus der Kaolin-Gruppe (siehe: Ullman's Encylopedia of Industrial Chemistry, 6. Auflage, 2000 elektronische Ausgabe, Kapitel 2 und Lehrbuch der Anorganischen Chemie, 91.-100. Auflage, 1985, Seiten 771 - 776) eingesetzt werden. Ganz besonderes bevorzugt wird Kaolinit eingesetzt.

Als Bindemittel für Schritt (I) werden bevorzugt Aluminiumverbindungen eingesetzt, die beim abschließenden Calzinierschritt zu mindestens 80% in γ-Al₂O₃ umgewandelt werden. Dazu gehören Aluminiumhydroxid und/oder Aluminiumoxid/-hydroxid. Als Aluminiumhydroxid kann sowohl das synthetische Al(OH)₃ oder das natürliche Hydrargillit [γ-Al(OH)_{3]} eingesetzt werden. Als Aluminiumoxid/-hydroxid [γ-Al(O)OH] werden bevorzugt Böhmit und/oder Pseudoböhmit eingesetzt. In einer besonderen Ausführungsform wird ein Gemisch aus Aluminiumhydroxid und/oder Aluminiumoxid/-hydroxid und γ-Al₂O₃ als Vorstufe eingesetzt.

Werden für die Herstellung des Katalysators natürlich vorkommende Mineralien eingesetzt, so können diese neben Silizium und/oder Aluminium auch die Elemente Titan, Eisen, Natrium und/oder Kalium in Spuren enthalten. Der Anteil an diesen Elementen liegt bevorzugt für Titan zwischen 0,1 und 1,0 Gew.-%, für Eisen zwischen 0,1 und 1,0 Gew.-%, für Kalium zwischen 0,1 und 5,0 Gew.-% und für Natrium zwischen 0,1 und 5,0 Gew.-%.

Die Homogenisierung des Gemisches nach Schritt (II) erfolgt bevorzugt in einem Kneter, Koller oder Extruder, z.B. für eine Dauer im Bereich von 10 bis 180 Minuten. Im kleineren Maßstab wird das Gemisch bevorzugt geknetet. Im industriellen, größeren Maßstab wird zur Homogenisierung bevorzugt gekollert. Bei der Homogenisierung wird bevorzugt bei Temperaturen im Bereich von ungefähr 10°C bis 100°C und unter Normaldruck oder leichtem überatmosphärischem Druck gearbeitet. Es wird solange homogenisiert, bis eine verformbare plastische Masse entstanden ist.

Die Formgebung nach Schritt (III) erfolgt bevorzugt durch Extrusion, Tablettieren, Brikettieren oder Pelletieren. Die Form der für das erfindungsgemäße Verfahren hergestellten Formkörper kann beliebig gewählt werden. Insbesondere sind unter anderem Kugeln, ovale Formen, Stränge oder Tabletten möglich.

Bevorzugt sind zylinderförmige Formkörper mit einem Durchmesser im Bereich von 0,5 bis 20 mm, bevorzugt im Bereich von 1 bis 10 mm, wobei das Länge : Durchmesser - Verhältnis insbesondere im Bereich von 0,5 bis 20, bevorzugt im Bereich von 1 bis 10, besonders bevorzugt im Bereich von 1,5 bis 5 liegt.

Besonders bevorzugt wird im Rahmen der vorliegenden Erfindung das Verformen durch Extrusion des gemäß Schritt II erhaltenen Gemischs durchgeführt.

Die Calcinierung nach Schritt (IV) wird bei Temperaturen im Bereich von bevorzugt 350 bis 750 °C und insbesondere von 450 bis 700 °C durchgeführt.

Die Calcinierung kann unter jeder geeigneten Gasatmosphäre erfolgen, wobei Luft und/oder Magerluft bevorzugt sind.

Weiter wird die Calcinierung bevorzugt in einem Muffelofen, einem Drehrohrofen und/oder einem Bandkalzinierofen durchgeführt, wobei die Calcinierungsdauer bevorzugt bei 1 h oder mehr, beispielsweise im Bereich von 1 bis 24 h oder im Bereich von 3 bis 12 h liegt. Demgemäß ist es im Rahmen des erfindungsgemäßen Verfahrens beispielsweise möglich, den Formkörper einmal, zweimal oder öfter für jeweils mindestens 1 h, wie beispielsweise für jeweils im Bereich von 3 bis 12 h, zu calcinieren, wobei die Temperaturen während eines Calcinierungsschrittes gleich bleiben oder kontinuierlich oder diskontinuierlich geändert werden können. Wird zweimal oder öfter calciniert, können sich die Calcinierungstemperaturen in den einzelnen Schritten unterscheiden oder gleich sein.

Nach dem Calcinierungsschritt kann das calcinierte Material beispielsweise zerkleinert werden. Dabei wird vorzugsweise ein Granulat oder Splitt mit einer Partikelgröße im Bereich von 0,1 bis 5 mm, insbesondere 0,5 bis 2 mm, erhalten.

Die erhaltenen Formkörper weisen Härten auf, die bevorzugt im Bereich von 2 bis 200 N (Newton), besonders bevorzugt im Bereich von 5 bis 150 N und ganz besonders bevorzugt mindestens 10 N betragen, z.B. im Bereich von 10 bis 100 N liegen.

Die obenstehend beschriebene Härte wurde im Rahmen der vorliegenden Erfindung an einem Apparat der Firma Zwick, Typ BZ2.5/TS1 S mit einer Vorkraft von 0,5 N, einer Vorkraftschubgeschwindigkeit von 10 mm/Min. und einer nachfolgenden Prüfgeschwindigkeit von 1,6 mm/Min. bestimmt. Das Gerät besaß einen festsitzenden Drehteller und einen frei beweglichen Stempel mit eingebauter Schneide von 0,3 mm Stärke. Der bewegliche Stempel mit der Schneide war mit einer Kraftmessdose zur Kraftaufnahme verbunden und bewegte sich während der Messung gegen den festsitzenden Drehteller hin, auf der der zu untersuchende Katalysatorformkörper lag. Das Prüfgerät wurde über einen Computer gesteuert, der die Messergebnisse registrierte und auswertete. Die erzielten Werte stellen den Mittelwert aus den Messungen zu jeweils mindestens 10 Katalysatorformkörpern dar.

Der Katalysator weist bevorzugt ein molares Al/Si-Verhältnis im Bereich von 0,1 bis 30, bevorzugt 1 bis 25, insbesondere bevorzugt 2 bis 20, auf. Die Angaben zum molaren Al/Si-Verhältnis im Formkörper beziehen sich auf den Gesamtgehalt an Al und Si.

Die spezifische Oberfläche des Katalysators, bestimmt gemäß DIN 66131 (BET), liegt bevorzugt bei mindestens 50 m²/g und insbesondere bevorzugt bei mindestens 100 m²/g. Beispielsweise liegt die spezifische Oberfläche im Bereich von 100 bis 250 m²/g und insbesondere im Bereich von 120 bis 200 m²/g.

Das Porenvolumen des Katalysators, bestimmt gemäß DIN 66134 (Hg-Porosimetrie), liegt bevorzugt bei mindestens 0,4 ml/g, besonders bevorzugt bei mindestens 0,6 ml/g. Beispielsweise liegt das Porenvolumen im Bereich von 0,4 bis 1,5 ml/g und insbesondere im Bereich von 0,6 bis 1,0 ml/g.

Der Katalysator enthält in einer bevorzugten Ausführungsform Spuren an Titan, Eisen, Natrium und/oder Kalium, jeweils in ionischer Form. Der Anteil dieser Elemente liegt für Titan im Bereich von ≥ 0,01 bis ≤ 0,35 Gew.-%, bevorzugt ≥ 0,05 bis ≤ 0,15 Gew.-%, für Eisen im Bereich von ≥0,01 bis ≤ 0,35 Gew.-%, bevorzugt ≥ 0,02 bis ≤ 0,10 Gew.-%, für Kalium im Bereich von ≥ 0,01 bis ≤ 1,75 Gew.-%, bevorzugt ≥ 0,10 bis ≤ 0,70 Gew.-%, und für Natrium im Bereich von ≥ 0,01 bis ≤ 1,75 Gew.-%, bevorzugt ≥ 0,10 bis ≤ 0,70 Gew.-%, jeweils bezogen auf das Formkörpergewicht.

Besonders bevorzugt enthält der Katalysator kein Phosphor in jedweder Oxidationsstufe.

### Regenerierung des Katalysators

In einer weiteren Ausführungsform des erfindungsgemäßen Verfahrens wird der Katalysator nach dem Einsatz unabhängig von seiner Form, z.B. nach Abnahme der Aktivität und/oder der Selektivität, durch ein Verfahren regeneriert, bei dem die Regenerierung durch gezieltes Abbrennen (bei z.B. einer Temperatur im Bereich von 350 bis 650 °C) der für die Deaktivierung verantwortlichen Beläge erfolgt. Dabei wird bevorzugt in einer Inertgasatmosphäre gearbeitet, die genau definierte Mengen an Sauerstoff oder Sauerstoff liefernden Substanzen enthält. Ein solches Regenerierungsverfahren ist unter anderem in der WO-A-98/55228 und der DE-A1-197 23 949 und insbesondere für Katalysatoren zur Herstellung von Methylaminen in der JP-08 157 428 und der EP-A-0118 193 beschrieben.

Nach der Regeneration sind die Aktivität und/oder die Selektivität des Katalysators, verglichen mit dem Zustand unmittelbar vor der Regeneration, erhöht.

Der zu regenerierende, im erfindungsgemäßen Verfahren eingesetzte Katalysator wird entweder in der Umsetzungsvorrichtung (Reaktor) oder in einem externen Ofen in einer Atmosphäre, die 0,1 bis ungefähr 20 Volumen-Anteile von Sauerstoff liefernden Substanzen, besonders bevorzugt 0,1 bis 20 Volumenanteile Sauerstoff, enthält, auf eine Temperatur im Bereich von 350 °C bis 800 °C, vorzugsweise von 400 °C bis 650°C und insbesondere von 425 °C bis 500 °C aufgeheizt. Dabei wird das Aufheizen vorzugsweise mit einer Aufheizrate von 0,1 °C/Min. bis 20 °C/Min., vorzugsweise von 0,3 °C/Min. bis 15 °C/Min. und insbesondere von 0,5 °C/Min. bis 10 °C/Min. durchgeführt. Das Aufheizen wird bevorzugt unter einer Inertatmosphäre durchgeführt.

Während der Regenerierung wird der Katalysator bis zu einer Temperatur aufgeheizt, bei der die sich dort befindlichen, meist organischen Beläge zu zersetzen beginnen, während gleichzeitig die Temperatur über den Sauerstoffgehalt geregelt wird und somit nicht derart ansteigt, dass es zu Schädigungen der Katalysatorstruktur oder des Reaktors kommt. Das langsame Erhöhen der Temperatur bzw. das Verweilen bei niedriger Temperatur durch Einstellen des entsprechenden Sauerstoffgehaltes und der entsprechenden Heizleistung ist bei hohen organischen Beladungen des zu regenerierenden Katalysators ein wesentlicher Schritt zur Verhinderung einer lokalen Überhitzung des Katalysators. Die Gasbelastung des Sauerstoff enthaltenden Regeneriergases ausgedrückt als GHSV (= gas hourly space velocity) liegt bevorzugt bei mehr als 50 Normliter pro Liter Katalysator und Stunde (= Nl/l(Kat)h), weiter bevorzugt bei mehr als 100 Nl/l(Kat)h und besonderes bevorzugt im Bereich zwischen 150 und 1000 Nl/l(Kat)h. (Nl = Normliter = auf Normalbedingungen umgerechnetes Volumen).

Sinkt die Temperatur des Abgasstroms am Reaktorausgang auf die Temperatur am Reaktoreingang trotz steigender Mengen an Sauerstoff oder Sauerstoff liefernden Substanzen im Gasstrom und/oder steigt die Konzentration an Sauerstoff im Reaktionsaustrag auf den Eingangswert an, so ist das Abbrennen der organischen Beläge beendet. Die Dauer der Behandlung beträgt bevorzugt jeweils 1 bis 72 h, vorzugsweise ungefähr 2 bis ungefähr 48 h und insbesondere ungefähr 3 bis ungefähr 24 Stunden.

Das anschließende Abkühlen des so regenerierten Katalysators wird bevorzugt so durchgeführt, dass das Abkühlen nicht zu schnell erfolgt, da sonst die mechanische Festigkeit des Katalysators negativ beeinflusst werden kann. Das Abkühlen wird bevorzugt unter einer Inertatmosphäre durchgeführt.

Es kann erforderlich sein, den Katalysator nach der durchgeführten Regeneration durch Calcinieren, wie oben beschrieben, einer Spülung mit Wasser und/oder verdünnten Säuren wie beispielsweise Salzsäure zu unterziehen, um die durch Verunreinigung der Edukte gegebenenfalls verbleibende anorganische Beladung des Katalysators (Alkalispuren etc.) zu entfernen. Anschließend kann eine erneute Trocknung und/oder ein erneutes Calcinieren des Katalysators durchgeführt werden.

In einer weiteren Ausführungsform des erfindungsgemäßen Verfahrens wird der zumindest teilweise deaktivierte Katalysator vor dem Aufheizen gemäß der Regenerationsprozedur mit einem Lösungsmittel im Umsetzungsreaktor oder in einem externen Reaktor gewaschen, um noch anhaftendes Wertprodukt zu entfernen. Dabei wird das Waschen so durchgeführt, dass zwar die jeweils am Katalysator anhaftenden Wertprodukte von diesem entfernt werden können, aber Temperatur und Druck nicht so hoch gewählt werden, dass die meist organischen Beläge ebenfalls entfernt werden. Vorzugsweise wird der Katalysator dabei mit einem geeigneten Lösungsmittel lediglich gespült. Somit eignen sich für diesen Waschvorgang alle Lösungsmittel, in denen sich das jeweilige Umsetzungsprodukt gut löst. Die benutzte Menge an Lösungsmittel sowie die Dauer des Waschvorgangs sind nicht kritisch. Der Waschvorgang kann mehrmals wiederholt und bei erhöhter Temperatur durchgeführt werden. Bei Verwendung von CO₂ als Lösungsmittel ist überkritischer Druck bevorzugt, ansonsten kann der Waschvorgang unter Normaldruck bzw. erhöhtem oder überkritischem Druck erfolgen. Nach der Beendigung des Waschvorgangs wird der Katalysator im Allgemeinen getrocknet. Obwohl der Trocknungsvorgang im allgemeinen unkritisch ist, sollte die Trocknungstemperatur die Siedetemperatur des zum Waschen verwendeten Lösungsmittels nicht zu stark übersteigen, um ein schlagartiges Verdampfen des Lösungsmittels in den Poren zu vermeiden, da dies zu Schädigungen des Katalysators führen kann.

Eine bevorzugte Ausführung des Herstellverfahrens besteht darin, dass das erfindungsgemäße Verfahren zur Synthese der primären Amine bei kontinuierlicher Durchführung bei der Regeneration des erfindungsgemäß verwendeten Katalysators nicht unterbrochen werden muss, um so den Verfahrensdurchsatz zu steigern. Dies kann durch die Verwendung von mindestens zwei parallel verschalteten Reaktoren erreicht werden, die wechselweise betrieben werden können.

Die Katalysatorregeneration kann derart durchgeführt werden, dass mindestens einer der parallel geschalteten Reaktoren aus der jeweiligen Reaktionsstufe abgekoppelt wird und der in diesem Reaktor enthaltene Katalysator regeneriert wird, wobei im Laufe des kontinuierlichen Verfahrens in jeder Stufe immer mindestens ein Reaktor zur Umsetzung der Edukte zur Verfügung steht.

### Beispiele

Für die folgenden Umsetzungen wurde ein amorpher Silica-Alumina-Katalysator, in Form von 1,0 bis 1,6 mm Splitt, mit einem molaren Verhältnis Al/Si = 25 eingesetzt, der zudem 0,04 Gew.-% Fe, 0,32 Gew.-% K und 0,09 Gew.-% Ti enthielt. Über Quecksilberporosimetrie (DIN 66134) wurde ein Porenvolumen von 0,56 ml/g und ein mittlerer Porendurchmesser von 0,01 µm bestimmt. Die BET-Oberfläche (DIN 66131) betrug 210 m²/g.

1)
Die Umsetzungen erfolgten in einem Rührautoklaven, wobei Alkohol und Katalysator vorgelegt wurden und anschließend Ammoniak aufgepresst wurde. Die Reaktionsmischung wurde unter Eigendruck auf die gewünschte Temperatur erhitzt und nach 12 h Reaktionszeit abgekühlt und auf Atmosphärendruck entspannt. Der Autoklaveninhalt wurde in Methanol aufgenommen und in einem Gaschromatographen analysiert. Die Angabe der Zusammensetzung des Austrags erfolgt in FID-Flächen%.

### Reaktionsbedingungen

| Versuch | R-OH | m(R-OH) | m(NH3) | m(Kat.) | T | p |
|---|---|---|---|---|---|---|
| 1 | tert.-BuOH | 24 g | 51 g | 4 g | 325 °C | 390 bar |
| 2 | Adaman-tanol | 25 g | 62 g | 20 g | 329°C | 450 bar |

### Analyse Reaktionsaustrag FID-Flächen%

| Versuch | R-OH | R-NH2 |
|---|---|---|
| 1 (R = tert.-Butyl) | 86,3 % | 12,0 % |
| 2 (R = 1-Adamantyl) | 79,2 % | 20.4 % |

2)
In einem Rohrreaktor (6 mm Innendurchmesser) wurden unter isothermen Bedingungen bei Temperaturen von 240-290 °C und Drücken von 35-100 bar Gemische aus Ammoniak und t-BuOH im molaren Verhältnis von 3-10 : 1 umgesetzt.

Genaue Reaktionsbedingungen, erreichte t-BuOH-Umsätze und t-Butylamin-Selektivitäten sind in nachfolgender Tabelle zusammengefasst.

| | | | | | | | |
|---|---|---|---|---|---|---|---|
| Kat.-Menge | g | 10 | 10 | 10 | 10 | 10 | 10 |
| nNH3 : nC4 | mol : mol | 10 | 3 | 10 | 10 | 10 | 10 |
| t-BuOH-Dosierung | g/h | 6 | 6 | 6 | 3 | 6 | 6 |
| NH3-Dosierung | g/h | 14 | 4 | 14 | 7 | 14 | 14 |
| Kat.-Belastung | g C4 / g Kat./h | 0,6 | 0,6 | 0,6 | 0,3 | 0,6 | 0,6 |
| p | bar | 100 | 100 | 100 | 100 | 100 | 35 |
| T | °C | 260 | 260 | 240 | 260 | 290 | 260 |
| | | | | | | | |
| Umsatz t-BuOH | % | 20 | 25 | 12 | 25 | 30 | 40 |
| Selektivität t-BuNH2 | % | 98 | 70 | 99 | 90 | 70 | 70 |

Die Ergebnisse verdeutlichen, dass man mit t-BuOH im geraden Duchgang höhere Umsätze fahren kann als bei iso-Buten/NH₃ - Umsetzungen an einem ZeolithKatalysator. Zudem benötigt man keine Hochdruckanlagen.

## Patentansprüche

1. Verfahren zur Herstellung eines primären Amins mit tertiärem alpha-C-Atom durch Umsetzung eines tertiären Alkohols mit Ammoniak in Gegenwart eines Heterogen-Katalysators, **dadurch gekennzeichnet, dass** man die Umsetzung in Gegenwart eines nicht-mikroporösen nicht-zeolithischen Alumosilikats als Katalysator durchführt, wobei das Alumosilikat ein molares Al/Si-Verhältnis im Bereich von 0,1 bis 30 aufweist.

2. Verfahren nach Anspruch 1 zur Herstellung eines primären Amins der Formel RR'R"C-NH₂ durch Umsetzung eines tertiären Alkohols der Formel RR'R"C-OH, wobei R, R' und R" organische Reste mit jeweils mindestens einem Kohlenstoffatom darstellen.

3. Verfahren nach Anspruch 1 oder 2, **dadurch gekennzeichnet, dass** als tertiärer Alkohol 2-Methyl-2-propanol, 2-Methyl-2-butanol oder 1-Adamantanol eingesetzt wird.

4. Verfahren nach einem der vorhergehenden Ansprüche, **dadurch gekennzeichnet, dass** der Katalysator ein molares Al/Si-Verhältnis im Bereich von 1 bis 25 aufweist.

5. Verfahren nach einem der vorhergehenden Ansprüche, **dadurch gekennzeichnet, dass** das Volumen der Poren des Katalysators größer als 0,3 ml/g beträgt.

6. Verfahren nach einem der Ansprüche 1 bis 4, **dadurch gekennzeichnet, dass** das Volumen der Poren des Katalysators im Bereich von 0,4 bis 1,5 ml/g liegt.

7. Verfahren nach einem der vorhergehenden Ansprüche, **dadurch gekennzeichnet, dass** der Katalysator eine BET-Oberfläche von ≥ 50 m²/g aufweist.

8. Verfahren nach einem der Ansprüche 1 bis 6, **dadurch gekennzeichnet, dass** der Katalysator eine BET-Oberfläche im Bereich von 100 bis 250 m²/g aufweist.

9. Verfahren nach einem der vorhergehenden Ansprüche, **dadurch gekennzeichnet, dass** der Katalysator neben Aluminium und Silicium auch im Bereich von 0,01 bis 1,75 Gew.-% Natrium, 0,01 bis 1,75 Gew.-% Kalium, 0,01 bis 0,35 Gew.-% Titan und/oder 0,01 bis 0,35 Gew.-% Eisen, jeweils in ionischer Form und jeweils bezogen auf das Gesamtgewicht des Katalysators, enthält.

10. Verfahren nach einem der vorhergehenden Ansprüche, **dadurch gekennzeichnet, dass** der Katalysator kein Phosphor enthält.

11. Verfahren nach einem der vorhergehenden Ansprüche, **dadurch gekennzeichnet, dass** der Katalysator als Formkörper eingesetzt wird, mit einem Länge zu Durchmesser - Verhältnis von ≥ 0,5.

12. Verfahren nach einem der vorhergehenden Ansprüche, **dadurch gekennzeichnet, dass** der Katalysator als Formkörper eingesetzt wird, mit einer Schneidhärte von ≥ 10 Newton (N).

13. Verfahren nach einem der vorhergehenden Ansprüche, **dadurch gekennzeichnet, dass** für die Herstellung des Katalysators Kaolin als Siliciumquelle verwendet wird.

14. Verfahren nach einem der vorhergehenden Ansprüche, **dadurch gekennzeichnet, dass** das Aluminiumoxid im Katalysator als gamma-Al₂O₃ vorliegt.

15. Verfahren nach dem vorhergehenden Anspruch, **dadurch gekennzeichnet, dass** als Vorstufe für das gamma-Al₂O₃ im Katalysator Aluminiumhydroxid und/oder Aluminiumoxid/-hydroxid (Böhmit oder Pseudoböhmit) verwendet wird.

16. Verfahren nach einem der vorhergehenden Ansprüche, **dadurch gekennzeichnet, dass** die Herstellung des Katalysators einen Extrusionsschritt oder einen Tablettierschritt beinhaltet.

17. Verfahren nach einem der vorhergehenden Ansprüche, **dadurch gekennzeichnet, dass** die Herstellung des Katalysators einen Calcinierschritt beinhaltet.

18. Verfahren nach dem vorhergehenden Anspruch, **dadurch gekennzeichnet, dass** der Calcinierschritt bei einer Temperatur im Bereich von 350 bis 750 °C und einer Dauer im Bereich von 1 bis 24 h durchgeführt wird.

19. Verfahren nach einem der vorhergehenden Ansprüche, **dadurch gekennzeichnet, dass** die Edukte Ammoniak und tertiärer Alkohol im molaren Verhältnis Ammoniak zu tertiärem Alkohol im Bereich von 0,6 bis 12 eingesetzt werden.

20. Verfahren nach einem der Ansprüche 1 bis 18, **dadurch gekennzeichnet, dass** die Edukte Ammoniak und tertiärer Alkohol im molaren Verhältnis Ammoniak zu tertiärem Alkohol im Bereich von 1 bis 3 eingesetzt werden.

21. Verfahren nach einem der vorhergehenden Ansprüche, **dadurch gekennzeichnet, dass** die Umsetzung bei einer Temperatur im Bereich von 220 bis 500 °C durchgeführt wird.

22. Verfahren nach einem der vorhergehenden Ansprüche, **dadurch gekennzeichnet, dass** die Umsetzung bei einem Absolutdruck im Bereich von 5 bis 400 bar durchgeführt wird.

23. Verfahren nach einem der Ansprüche 1 bis 21, **dadurch gekennzeichnet, dass** die Umsetzung bei einem Absolutdruck im Bereich von 10 bis 250 bar durchgeführt wird.

24. Verfahren nach einem der vorhergehenden Ansprüche, **dadurch gekennzeichnet, dass** die Katalysatorbelastung im Bereich von 0,1 bis 2,0 kg(tertiärer Alkohol) • kg(Katalysator) ⁻¹ • h⁻¹ liegt.

25. Verfahren nach einem der vorhergehenden Ansprüche, **dadurch gekennzeichnet, dass** eine Regenerierung des eingesetzten Katalysators durch gezieltes Abbrennen der für die Deaktivierung verantwortlichen Beläge erfolgt.

## Claims

1. A process for preparing a primary amine with a tertiary alpha-carbon atom by reacting a tertiary alcohol with ammonia in the presence of a heterogeneous catalyst, which comprises performing the reaction in the presence of a non-microporous, non-zeolitic aluminosilicate as a catalyst, where the aluminosilicate has a molar Al/Si ratio in the range from 0.1 to 30.

2. The process according to claim 1 for preparing a primary amine of the formula RR'R"C-NH₂ by reacting a tertiary alcohol of the formula RR'R"C-OH where R, R' and R" are each organic radicals having in each case at least one carbon atom.

3. The process according to claim 1 or 2, wherein the tertiary alcohol used is 2-methyl-2-propanol, 2-methyl-2-butanol or 1-adamantanol.

4. The process according to any one of the preceding claims, wherein the catalyst has a molar Al/Si ratio in the range from 1 to 25.

5. The process according to any one of the preceding claims, wherein the volume of the pores of the catalyst is greater than 0.3 ml/g.

6. The process according to any one of claims 1 to 4, wherein the volume of the pores of the catalyst is in the range from 0.4 to 1.5 ml/g.

7. The process according to any one of the preceding claims, wherein the catalyst has a BET surface area of ≥ 50 m²/g_{.}

8. The process according to any one of claims 1 to 6, wherein the catalyst has a BET surface area in the range from 100 to 250 m²/g.

9. The process according to any one of the preceding claims, wherein the catalyst, as well as aluminum and silicon, also comprises in the range from 0.01 to 1.75% by weight of sodium, from 0.01 to 1.75% by weight of potassium, from 0.01 to 0.35% by weight of titanium and/or from 0.01 to 0.35% by weight of iron, in each case in ionic form and based in each case on the total weight of the catalyst.

10. The process according to any one of the preceding claims, wherein the catalyst does not comprise any phosphorus.

11. The process according to any one of the preceding claims, wherein the catalyst is used in the form of a shaped body with a length to diameter ratio of ≥ 0.5.

12. The process according to any one of the preceding claims, wherein the catalyst is used in the form of a shaped body with a cutting hardness of ≥ 10 newtons (N).

13. The process according to any one of the preceding claims, wherein kaolin is used as the silicon source for the preparation of the catalyst.

14. The process according to any one of the preceding claims, wherein the aluminum oxide in the catalyst is present in the form of gamma-Al₂O₃.

15. The process according to the preceding claim, wherein the precursor used for the gamma-Al₂O₃ in the catalyst is aluminum hydroxide and/or aluminum oxide/hydroxide (boehmite or pseudoboehmite).

16. The process according to any one of the preceding claims, wherein the preparation of the catalyst includes an extrusion step or a tableting step.

17. The process according to any one of the preceding claims, wherein the preparation of the catalyst includes a calcination step.

18. The process according to the preceding claim, wherein the calcination step is carried out at a temperature in the range from 350 to 750°C and for a duration in the range from 1 to 24 h.

19. The process according to any one of the preceding claims, wherein the ammonia and tertiary alcohol reactants are used in a molar ammonia to tertiary alcohol ratio in the range from 0.6 to 12.

20. The process according to any one of claims 1 to 18, wherein the ammonia and tertiary alcohol reactants are used in a molar ammonia to tertiary alcohol ratio in the range from 1 to 3.

21. The process according to any one of the preceding claims, wherein the reaction is performed at a temperature in the range from 220 to 500°C.

22. The process according to any one of the preceding claims, wherein the reaction is carried out at an absolute pressure in the range from 5 to 400 bar.

23. The process according to any one of claims 1 to 21, wherein the reaction is performed at an absolute pressure in the range from 10 to 250 bar.

24. The process according to any one of the preceding claims, wherein the catalyst hourly velocity is in the range from 0.1 to 2.0 kg (tertiary alcohol) • kg (catalyst)⁻¹ • h⁻¹.

25. The process according to any one of the preceding claims, wherein the catalyst used is regenerated by controlled burning-off of the deposits responsible for the deactivation.

## Revendications

1. Procédé pour la préparation d'une amine primaire avec un atome de carbone tertiaire en position alpha par transformation d'un alcool tertiaire avec de l'ammoniac en présence d'un catalyseur hétérogène, **caractérisé en ce qu'**on réalise la transformation en présence d'un aluminosilicate non microporeux, non zéolithique comme catalyseur, l'aluminosilicate présentant un rapport molaire Al/Si dans la plage de 0,1 à 30.

2. Procédé selon la revendication 1 pour la préparation d'une amine primaire de formule RR'R"C-NH₂ par transformation d'un alcool tertiaire de formule RR'R"C-OH, où R, R' et R" représentent des radicaux organiques comprenant à chaque fois au moins un atome de carbone.

3. Procédé selon la revendication 1 ou 2, **caractérisé en ce qu'**on utilise comme alcool tertiaire du 2-méthyl-2-propanol, du 2-méthyl-2-butanol ou du 1-adamantanol.

4. Procédé selon l'une quelconque des revendications précédentes, **caractérisé en ce que** le catalyseur présente un rapport molaire Al/Si dans la plage de 1 à 25.

5. Procédé selon l'une quelconque des revendications précédentes, **caractérisé en ce que** le volume des pores du catalyseur est supérieur à 0,3 ml/g.

6. Procédé selon l'une quelconque des revendications 1 à 4, **caractérisé en ce que** le volume des pores du catalyseur se situe dans la plage de 0,4 à 1,5 ml/g.

7. Procédé selon l'une quelconque des revendications précédentes, **caractérisé en ce que** le catalyseur présente une surface spécifique BET ≥ 50 m²/g_{.}

8. Procédé selon l'une quelconque des revendications 1 à 6, **caractérisé en ce que** le catalyseur présente une surface spécifique BET dans la plage de 100 à 250 m²/g.

9. Procédé selon l'une quelconque des revendications précédentes, **caractérisé en ce que** le catalyseur contient, outre de l'aluminium et du silicium, également dans la plage de 0,01 à 1,75% en poids de sodium, 0,01 à 1,75% en poids de potassium, 0,01 à 0,35% en poids de titane et/ou 0,01 à 0,35% en poids de fer, à chaque fois sous forme ionique et à chaque fois par rapport au poids total du catalyseur.

10. Procédé selon l'une quelconque des revendications précédentes, **caractérisé en ce que** le catalyseur ne contient pas de phosphore.

11. Procédé selon l'une quelconque des revendications précédentes, **caractérisé en ce que** le catalyseur est utilisé sous forme de corps façonné présentant un rapport longueur à diamètre ≥ 0,5.

12. Procédé selon l'une quelconque des revendications précédentes, **caractérisé en ce que** le catalyseur est utilisé sous forme de corps façonné, présentant une dureté à la coupe ≥ 10 Newton (N).

13. Procédé selon l'une quelconque des revendications précédentes, **caractérisé en ce qu'**on utilise du kaolin comme source de silicium pour la préparation du catalyseur.

14. Procédé selon l'une quelconque des revendications précédentes, **caractérisé en ce que** l'oxyde d'aluminium se trouve sous forme de gamma-Al₂O₃ dans le catalyseur.

15. Procédé selon la revendication précédente, **caractérisé en ce qu'**on utilise comme précurseur pour le gamma-Al₂O₃ dans le catalyseur de l'hydroxyde d'aluminium et/ou de l'oxyde/hydroxyde d'aluminium (boehmite ou pseudoboehmite).

16. Procédé selon l'une quelconque des revendications précédentes, **caractérisé en ce que** la préparation du catalyseur comporte une étape d'extrusion ou une étape de compression en comprimés.

17. Procédé selon l'une quelconque des revendications précédentes, **caractérisé en ce que** la préparation du catalyseur comporte une étape de calcination.

18. Procédé selon la revendication précédente, **caractérisé en ce que** l'étape de calcination est réalisée à une température dans la plage de 350°C à 750°C et à une durée dans la plage de 1 à 24 heures.

19. Procédé selon l'une quelconque des revendications précédentes, **caractérisé en ce que** les produits de départ ammoniac et alcool tertiaire sont utilisés dans un rapport molaire d'ammoniac à alcool tertiaire dans la plage de 0,6 à 12.

20. Procédé selon l'une quelconque des revendications 1 à 18, **caractérisé en ce que** les produits de départ ammoniac et alcool tertiaire sont utilisés dans un rapport molaire d'ammoniac à alcool tertiaire dans la plage de 1 à 3.

21. Procédé selon l'une quelconque des revendications précédentes, **caractérisé en ce que** la transformation est réalisée à une température dans la plage de 220 à 500°C.

22. Procédé selon l'une quelconque des revendications précédentes, **caractérisé en ce que** la transformation est réalisée à une pression absolue dans la plage de 5 à 400 bars.

23. Procédé selon l'une quelconque des revendications 1 à 21, **caractérisé en ce que** la transformation est réalisée à une pression absolue dans la plage de 10 à 250 bars.

24. Procédé selon l'une quelconque des revendications précédentes, **caractérisé en ce que** la charge du catalyseur se situe dans la plage de 0,1 à 2,0 kg(alcool tertiaire) * kg (catalyseur)⁻¹ * h⁻¹.

25. Procédé selon l'une quelconque des revendications précédentes, **caractérisé en ce qu'**une régénération du catalyseur utilisé a lieu par la calcination ciblée des recouvrements responsables de la désactivation.
